# EUROPEAN PATENT SPECIFICATION

(11) **EP 1 993 670 B1**
(45) Date of publication and mention of the grant of the patent: **18.04.2012**
(21) Application number: 06850378.8
(22) Date of filing: 30.12.2006
(51) Int. Cl.: A61M 37/00

(54) **ACOUSTIC PRESSURE WAVE APPLICATOR SYSTEM WITH CONDUCTION PAD**
SYSTEM MIT FÜHRUNGSPAD ZUR ANWENDUNG AKUSTISCHER DRUCKWELLEN
SYSTÈME APPLICATEUR D'ONDES DE PRESSION ACOUSTIQUE À COMPRESSE DE CONDUCTION

(30) Priority: 30.12.2005 US 754648 P
(43) Date of publication of application: 26.11.2008
(73) Proprietor: Sanuwave, Inc., Alpharetta, GA 30022 (US)
(72) Inventor: MENZI, Manfred, CH-9470 Buchs (CH); SCHOCK, Frank, 78467 Konstanz (DE); BRAUCHLI, Rene, CH-8573 Alterswilen (CH)
(74) Representative: Raffay & Fleck
(86) International application number: PCT/US2006/062771
(87) International publication number: WO 2007/108854

(56) References cited:
- WO-A2-03/061726
- US-A1- 2004 220 456
- US-A1- 2005 215 901
- US-B2- 6 723 077

## Description

### Technical Background

The present invention relates to a system for applying acoustic shock waves, in therapeutic treatments of humans and animals, wherein one or more pads are utilized in the shock wave applicator system. Such a device is disclosed in the WO 03061726.

### Background Art

In conventional acoustic shock wave treatments, extracorporeal shock wave technologies such as electrohydraulic shock wave generation (or spark gap method), electromagnetic shock wave generation, piezoelectric shock wave generation, and the like, are used to direct acoustic pressure waves for focused treatment of desired target areas of humans and animals. Typically, shock waves are focused for penetration into the body wherein the shock wave converges with maximum intensity at the target area.

In other acoustic pressure wave applications and devices, such as disclosed in U.S. Pat. Pub. No. 2006/0100549 to Schultheiss et al., focused, unfocused, divergent and planar waves may also be used to administer shock waves to target areas. Such target areas may include skin, wounds, scars and the like, as disclosed in International Pub. No. W02005/075020 to Meirer et al.. Meirer et al. also suggests that gel pads and other sterile barriers may be used in conjunction with shock wave applicators to maintain sterility while treating wounds and skin.

With respect to ultrasound devices, as distinguished from acoustic pressure wave generators, such as the shock wave producing technologies, ultrasound flex-gel standoffs are known for providing a molding function to better couple an ultrasound head to contoured surfaces of a body.

### Disclosure of the Invention

The present invention improves upon the use of pads as sterility barriers or as ultrasound standoffs by providing a system according to claim 1 wherein acoustic pressure waves can be utilized with one or more pads to (1) provide acoustic pressure wave focusing elements and/or (2) administer and enhance the absorption of bioactive substances with respect to a desired target area.

### Brief Description of the Drawings

FIG. 1 is a schematic cross-sectional diagram depicting an acoustic pressure wave generator and coupler coupled to a pad with application to a focal point located in a target area in an embodiment of the present invention.
FIG. 2 is a schematic cross-sectional diagram depicting an acoustic pressure wave generator and coupler coupled to a pad with application to a focal point in the pad with divergent waves applied at a target area in an embodiment of the present invention.
FIG. 3 is a schematic cross-sectional diagram depicting an acoustic pressure wave generator and coupler coupled to a pad with application of planar waves to a target area in an embodiment of the present invention.
FIG. 4 is a schematic cross-sectional diagram depicting an acoustic pressure wave generator and coupler coupled to a pad with application to a focal point in the coupler with divergent waves applied at a target area in an embodiment of the present invention.
FIG. 5 is a schematic cross-sectional diagram depicting an acoustic pressure wave generator and coupler coupled to a pad with application through a pad including a blocking element in an embodiment of the present invention.
FIG. 6 is a schematic cross-sectional diagram depicting an acoustic pressure wave generator and coupler coupled to a pad including a screening element with waves applied at a target area in an embodiment of the present invention.
FIG. 7 is a schematic cross-sectional diagram depicting an acoustic pressure wave generator and coupler coupled to a pad including a sterile barrier element covering a shock wave applicator head in an embodiment of the present invention.
FIG. 8 is a schematic cross-sectional diagram depicting an acoustic pressure wave generator and coupler coupled to a pad including a sterile barrier element covering a shock wave applicator head and further covering a surrounding area of the target area in an embodiment of the present invention.
FIG. 9 is a schematic cross-sectional diagram depicting an acoustic pressure wave generator and coupler coupled to a pad wherein the coupler is adjustable in an embodiment of the present invention.

### Best Mode for Carrying Out the Invention

Embodiments of the invention will be described with reference to the accompanying drawings and figures wherein like numbers represent like elements throughout. Further, it is to be understood that the phraseology and terminology used herein is for the purpose of description and should not be regarded as limiting. The use of "including", "comprising", or "having" and variations thereof herein is meant to encompass the items listed thereafter and equivalents thereof as well as additional items. The terms "mounted", "connected", and "coupled" are used broadly and encompass both direct and indirect mounting, connecting and coupling. Further, "connected" and "coupled" are not restricted to physical or mechanical connections or couplings.

While embodiments of the invention are described with respect to therapeutic and medical activities, it will be appreciated that the invention encompasses a wide variety of non-medical uses, including biotechnology, chemical, industrial, geophysical, material sciences, agricultural, electronics, genetics and other applications.

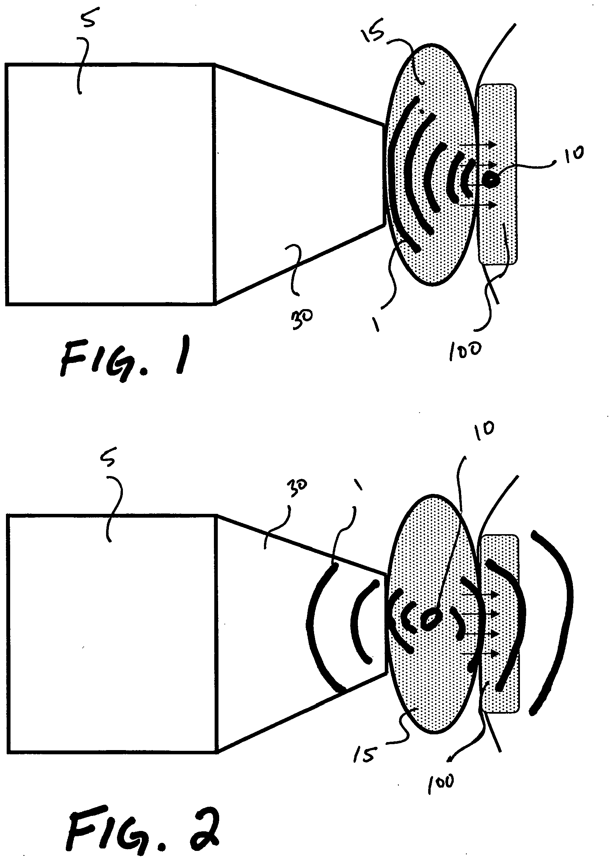

Referring to FIGS. 1-4, the present invention provides a system for applying acoustic pressure waves to a target area 100 with an acoustic conduction pad 15 that includes an absorbable and bioactive substance such as an ointment, unguent, medicament, hydrator, antibiotic, antiseptic, sterilizer, cosmetic, anesthetic, gel and the like, that produces a biological effect in the target area 100. Such substances may be coated on a conduction pad 15, dispersed or dispensed from the pad 15 and/or integral to a pad 15, including, but not limited to, in a molecular or macromolecular matrix. In various embodiments of the invention, pads may be sponge-like with absorbable substances dispensed from mechanical pressure, may be aqueous gel-type pads, may be containment pads with perforations or holes permitting dispensing of an absorbable substance, may have permeable membranes, may comprise a material in which interstitial spaces are embedded with a dispersible substance, and the like.

A shock wave generator 5, such as electrohydraulic shock wave generation (or spark gap method), electromagnetic shock wave generation, piezoelectric shock wave generation, radial pressure wave generation (such as pneumatically generated shock waves), and the like, produce a shock wave 1, and typically a plurality of shock waves of desired frequency and intensity, that is conducted through a coupler 30. A coupler 30 may include one or more acoustic pressure waves conductive components, such as a firm or flexible membrane or window with a conductive medium. An acoustic wave conduction pad 15 couples to the coupler 30. In embodiments of the invention, a coupler includes a membrane or window integrated in a therapy head for conduction of acoustic pressure waves into a target area 100 of an animal or human body, and couples with a conductive pad 15 that includes an absorbable bioactive substance.

Exemplary substances (illustrative and not exclusive), including mixtures thereof, are: cosmetics (e.g. herbs essence), a pain relieving medicament (e.g. diclofenac) or other medicaments, a local anaesthetic (e.g. procain, lidocain), a wound disinfection agent (e.g. phenoxyethanol, dexpanthenol, jodine), antimicrobial, antibiotic and other sterilizers, an ultra sonic gel, water and hydrators, ointment, unguent and gels.

The pad 15 can store these substances in a molecular or a macromolecular matrix. Examples include sponge-like structures on the macromolecular basis or gel-like structures on the molecular basis. A gel is a fine dispersive system comprising at least a firm and a liquid phase, representing a colloid. The firm phase forms thereby a sponge-like, three dimensional network, whose pores are filled out by a liquid and/or a fluid. This material can be also a compound material out of these two types of matrixes.

The storage matrix is not required to be fixed directly on the top of the coupler 30. It could be shaped around the coupler 30, the part of the pad 15 permeable for sonic waves, or the part of the pad 15 maintaining the distance between the target area 100 and the coupler 30. In some embodiments, pads, with our without an absorbable substance, may be comprised of a plurality of compositions or materials to provide a density gradient of the pad. The density gradient may be configured for the desired application of shock waves into the body and/or for the most effective absorption of an absorbable substance into the target area 100.

In embodiments of the invention, parts of a therapy head or pad having contact with the treated patient (animal or human being), are coated with a special, appropriate substance. This substance may include silver or other antiseptics or lubricants such as teflon and others.

In embodiments of the invention, a plurality of absorbable substances may be combined in a pad 15 for application to a target area 100.

One or more pads 15 may be coupled to coupler 30 in configurations in which the pads 15 in various embodiments, including but not limited to, integrated, strapped, clipped, snapped, tied, hung, slotted, adhered (such as adhesives), and/or independently held in place by force or friction. Other coupling configurations will be appreciated as within the skill of those in the art. Pads 15 are preferably disposable, but may be reusable, such as if sterilized and replenished with a dispersible or dispensable substance.

Shock waves 1 facilitate the absorption and bioactive effect of such substances propelled into the target area 100 of a body, such as skin or a wound. In other embodiments, target area 100 may include tissues accessible during surgery, the mouth and gums, body membranes, organs, nerves, spinal cord, blood vessels, ischemic regions, and the like for which desired absorption of a bioactive substance administered via shockwaves from a conduction pad 15 is desired. In further embodiments, bioactive substances in conjunction with application of shock waves 1 to a target area 100 may stimulate or enhance cellular and genetic activities and expression in tissues both in vivo and in vitro.

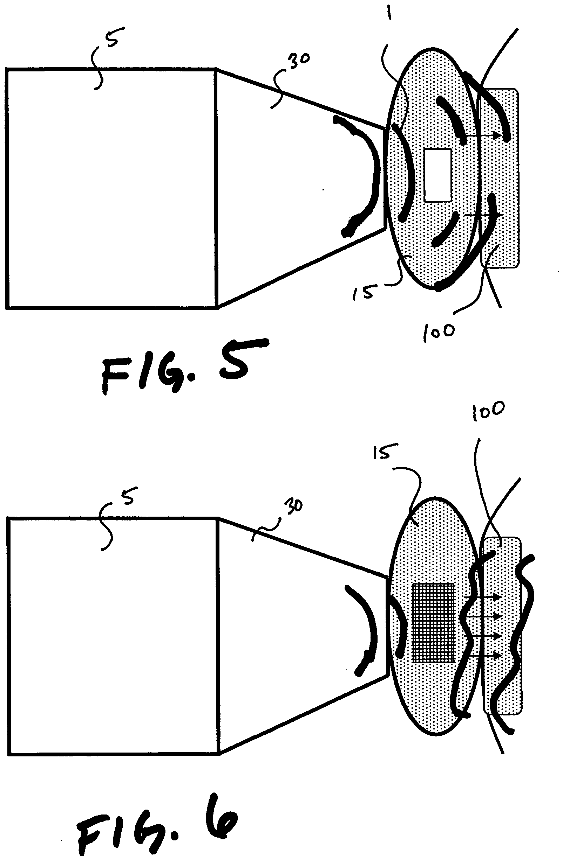

A pad 15 or spacer can have one or more functions from of the following non-exclusive list of functions: (i) maintain a distance between the treated area and the therapy head; (ii) act as an acoustical lens influencing the sonic path of the shock wave by form and density (e.g. if having a certain shape and/or if made out of a material with a different density than the material the shock wave is emitted from); and (iii) enhance or dampen the sonic wave (e.g. by introducing some metal foil or other materials into the pad structure) (FIGS. 5 and 6). A plurality of pads 15 may be used to adjust the location of a convergence focal point 10 within a coupler 30, in a particular pad 15 or at the target area 100.

Referring to FIGS. 1-9, it will be appreciated that a focusing element (not shown) is typically utilized with a shock wave generator 5 and coupler 30. A shock wave focusing element may include a reflector of a wide variety of shapes, an acoustic lens (such as convex or concave) or a wave-directing element (e.g. a partial conical segment) used with the acoustic pressure wave generator 5. In embodiments where shock waves are focused, such as shown in FIGS. 1, 2 and 4, the shock wave reaches maximum intensity at a convergence focal point 10.

Referring to FIG. 1, an embodiment of the invention is shown wherein the convergence focal point 10 is positioned in the target area 100, as the waves 1 converge from the coupler 30 through the pad 15 and propel one or more bioactive substances in the pad for absorption by the target area 100.

Referring to FIG. 2, in an alternative embodiment, the convergence focal point 10 is positioned within pad 15 and the maximum intensity of the shock wave 1 propels one or more bioactive substances in the pad for absorption by the target area 100. In this embodiment, a divergent focal field follows convergence point 10 and can provide controlled penetration into the target area 100 as the intensity of the divergent focal field is reduced distal to the convergence point 10. In such embodiment, pain or unwanted penetration depth may be reduced or avoided in comparison to focusing into a target area 100.

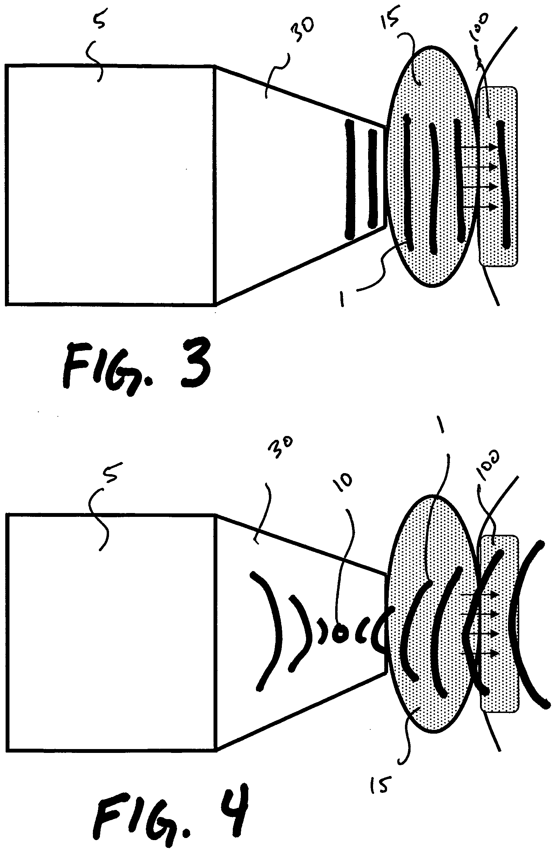

Similarly, FIG. 4, depicts an embodiment wherein the target area 100 is distal from focal point 10. In this embodiment, the focal point 10 is positioned in the coupler 30, and a divergent focal field passes through pad 15 to conduct a bioactive substance into the target area 100, which also remains in the divergent focal field.

In other embodiments, unfocused, planar, nearly planar and other divergent wave patterns, such as described in U.S. Pat. Pub. No. 2006/0100549 to Schultheiss et al., may be utilized with one or more focusing elements to achieve desired intensity and penetration with respect to pad 15 and the target area 100. FIG. 3 shows one example wherein planar waves 1 are conducted through pad 15 propelling bioactive substance(s) into target area 100.

In some embodiments a focusing element may be adjustable in position with respect to the point of acoustic pressure wave generation in the shock wave generator to place the focal point in alternative positions and change the intensity, field scope and penetration depth of shock waves. In further embodiments a focusing element may comprise a variety of shapes to produce focused, unfocused, planar or divergent waves used in conjunction with a pad including an absorbable substance.

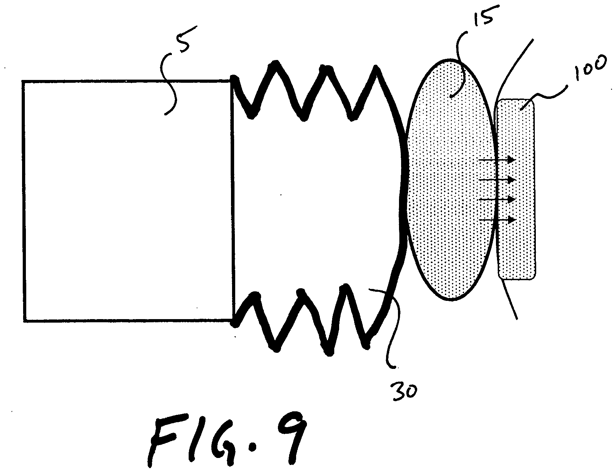

Referring to FIG. 9, in another embodiment, a coupler 30 comprises an accordion-like wall that is flexible and adjustable lengthwise for adjusting a shock wave convergence focal point and controlling penetration depth of the acoustic pressure waves into and beyond the target area to achieve desired absorption effects of the substance from the pad. In other embodiments, the coupler may be stretched or partially elongated, such as by mechanical reconfiguration or flexible adjustment, to adjust the location of a convergence focal point 10 within the coupler and the subsequent scope of a divergent focal field continuing thereform.

In further embodiments a coupler 30 may encompass modular pads or spacers, including the use of a plurality of pads or "stacking" configurations to achieve desired intensity of the shock waves at the target area 100.

In other embodiments, a pad or pads 15 may be used, with or without an absorbable substance, as an acoustic focusing or unfocusing (acoustic diffuser) element to achieve desired intensity of penetration of shock waves at and outside the target area 100. In such embodiments the pad may be shaped, such as convex or concave, including combinations thereof, to generate focused, unfocused, planar and divergent shock waves and focal fields in conjunction with a shock wave coupler 30. Focusing elements, such as reflectors and acoustic lenses, as previously described, may be used in conjunction with a pad 15 also functioning as an acoustic focusing or unfocusing element.

In some embodiments of the invention, a pad, with or without an absorbable composition, may include physical devices or materials for manipulating the focus of shock waves applied to a target area. In one embodiment, shown in FIG. 5, a wave buffer, obstruction or blocker material 70, such as a dense object of a pre-selected shape for a particular application, may be positioned in a pad to block or buffer the shock waves striking the object, while shock waves pass through non-impeded areas of the pad. In other embodiments, shown in FIG. 6, a screening material 80 may be included in the pad so that an acoustic pressure wave is diffused as it passes through the screening material to achieve a desired intensity of the application to a target area 100. In other embodiments, the pad may include a particular material or composition or combinations thereof that enhance or buffer or obstruct the penetration of shock waves with respect to one or more areas of a pad. Such compositions or combinations may also be used to affect the focus of the waves administered through the pad 15.

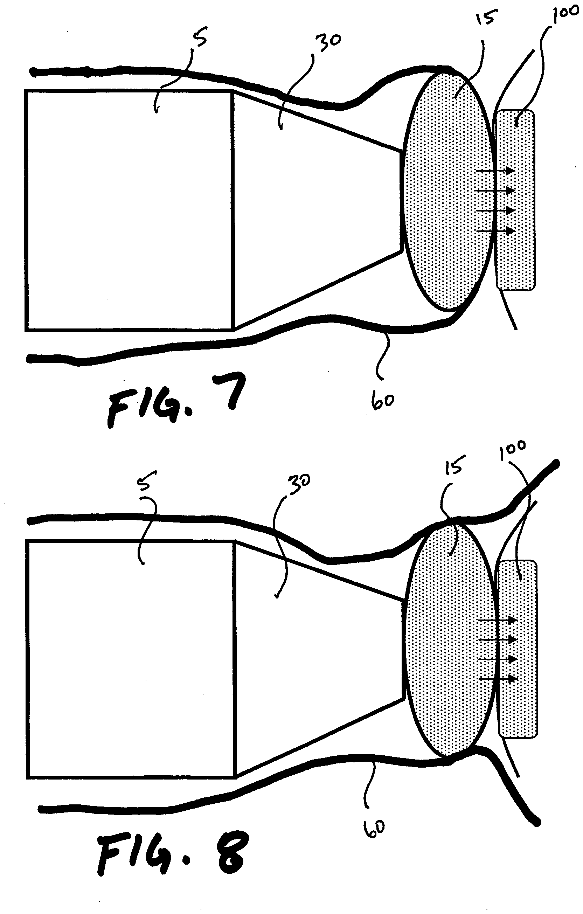

Referring to FIGS. 7 and 8, in embodiments of the invention, a pad 15, with or without an absorbable substance, includes an integral or attachable/removable sterility barrier, such as a sterile wrapping that includes an acoustic conductor pad portion 15 that may be positioned for coupling with a shock wave coupler 30 (e.g. membrane or window) and a "wrap" portion 60 that extends for covering surrounding areas, such as a therapy head throat or handle (FIG. 7), areas surrounding a target area 100 or both the therapy device and the surrounding area (FIG. 8)

In other embodiments, the present invention may be used in applying acoustic pressure waves through pads in biotechnology applications and research, including the stimulation or targeting of cells, microbes, molecules, viruses, genetic material, microbes, bacteria, microorganisms, plant tissue and the like. In alternative embodiments, the present invention may be utilized in variety of chemical, industrial, geophysical, semiconductor and electronics applications, including the development of materials better able to withstand vibration and other forces that lead to cracking, shattering, weakening, degradation, movement, deformation and the like.

Although the invention herein has been described with reference to particular embodiments, it is to be understood that these embodiments are merely illustrative of the principals and applications of the present invention. Accordingly, while the invention has been described with reference to the structures and processes disclosed, it is not confined to the details set forth, but is intended to cover such modifications or changes as may fall within the scope of the following claims.

## Claims

1. An acoustic pressure wave applicator system for applying acoustic shock waves to facilitate absorption of an absorbable substance into a target area comprising:
an acoustic shock wave generator (5) selected from the group consisting of an electrohydraulic shock wave generator, an electromagnetic shock wave generator, radial pressure wave generator and a piezoelectric shock wave generator;
a coupler (30) including a conductive medium for conduction of acoustic shock waves from the acoustic shock wave generator to a target area; and
an acoustic conduction pad (15) coupled to the coupler including a dispersible bioactive substance absorbed by the target area.

2. The applicator system according to claim 1 wherein the bioactive substance is selected from the group consisting of an ointment, unguent, cosmetic, medicament, hydrator, anesthetic, antiseptic, antibiotic and sterilizer.

3. The system according to one of the preceding claims, wherein the pad is positioned for conduction of acoustic pressure waves in a divergent focal field to a target area including skin.

4. The system according to one of the preceding claims, wherein the pad includes a wave screening or blocking material.

5. The system according to one of the preceding claims, wherein the pad includes a sterile wrap portion covering a throat of a shock wave applicator housing the coupler.

6. The system according to one of the preceding claims, wherein the pad includes a gradient of a plurality of materials.

7. The system according to one of claims 1, 2 and 4-6, wherein the pad is shaped to focus an acoustic pressure wave.

8. The system according to one of claims 1, 2 and 4-6, wherein the pad is shaped to diffuse an acoustic pressure wave.

9. The system according to one of the preceding claims, wherein the pad include one or more compositions affecting the focusing characteristics of an acoustic pressure wave.

10. The system according to one of claims 1, 2, 4-6 and 9, wherein the coupler is configured to focus a shock wave to a convergence focal point (10) in the pad.

11. The system according to one of claims 1, 2, 4-6 and 9, wherein the coupler is configured to focus a shock wave to a convergence focal point in the target area.

12. The system according to one of the preceding claims, further comprising a focusing element adjustable coaxially for controlling penetration depth of the acoustic pressure waves into and beyond the target area.

13. The system according to one of the preceding claims, wherein the coupler includes one or more spacers for controlling penetration depth and intensity of the acoustic pressure waves with respect to the target area.

14. The system according to one of the preceding claims, wherein the coupler includes an attachment for securing and coupling the pad to the coupler.

15. The system according to one of the preceding claims, wherein the conduction pad includes a matrix of pores storing the bioactive substance as a colloidal gel.

## Patentansprüche

1. Applikatorsystem für akustische Druckwellen zum Applizieren akustischer Stoßwellen, um die Absorption einer absorbierbaren Substanz in einen Zielbereich zu unterstützen, wobei das System Folgendes umfasst:
einen Generator akustischer Stoßwellen (5), ausgewählt aus einer Gruppe, die aus einem Generator elektrohydraulischer Stoßwellen, einem Generator elektromagnetischer Stoßwellen, einem Generator von Radialdruckwellen und einem Generator piezoelektrischer Stoßwellen besteht;
ein Kupplungselement (30), das ein leitfähiges Medium zum Leiten akustischer Stoßwellen vom Generator akustischer Stoßwellen zu einem Zielbereich enthält; und
eine akustisch leitfähige Unterlage (15), die mit dem Kupplungselement verbunden ist und eine vom Zielbereich zu absorbierende dispersible, bioaktive Substanz enthält.

2. Applikatorsystem nach Anspruch 1, wobei die bioaktive Substanz aus einer Gruppe ausgewählt wird, zu der ein Balsam, eine Salbe, ein Kosmetikum, ein Medikament, ein Hydrator, ein Anästhetikum, ein Antiseptikum, ein Antibiotikum und ein Sterilisator gehören.

3. System nach einem der vorstehenden Ansprüche, wobei die Unterlage so positioniert ist, dass akustische Druckwellen in einem divergenten Fokalfeld zu einem Zielbereich, einschließlich Haut, geleitet werden.

4. System nach einem der vorstehenden Ansprüche, wobei die Unterlage ein Material enthält, das Wellen abschirmt oder blockiert.

5. System nach einem der vorstehenden Ansprüche, wobei die Unterlage einen Teil mit einer sterilen Umwickelung umfasst, die einen Hals eines Stoßwellenapplikators verdeckt, in dem das Kupplungselement untergebracht ist.

6. System nach einem der vorstehenden Ansprüche, wobei die Unterlage einen Gradient einer Vielzahl von Materialien umfasst.

7. System nach einem der Ansprüche, 1, 2 und 4-6, wobei die Unterlage so gestaltet ist, dass sie eine akustische Druckwelle fokussiert.

8. System nach einem der Ansprüche, 1, 2 und 4-6, wobei die Unterlage so gestaltet ist, dass sie eine akustische Druckwelle diffundiert.

9. System nach einem der vorstehenden Ansprüche, wobei die Unterlage eine oder mehrere Kompositionen umfasst, die die fokussierenden Eigenschaften einer akustischen Druckwelle beeinflussen.

10. System nach einem der Ansprüche 1, 2, 4-6 und 9, wobei das Verbindungselement so konfiguriert ist, dass es eine Stoßwelle auf einen Konvergenz-Fokalpunkt (10) in der Unterlage fokussiert.

11. System nach einem der Ansprüche 1, 2, 4-6 und 9, wobei das Verbindungselement so konfiguriert ist, dass es eine Stoßwelle auf einen Konvergenz-Fokalpunkt im Zielbereich fokussiert.

12. System nach einem der vorstehenden Ansprüche, das darüber hinaus ein Fokussierelement umfasst, das koaxial einstellbar ist, um die Penetrationstiefe der akustischen Druckwellen in den Zielbereich und über den Zielbereich hinaus zu steuern.

13. System nach einem der vorstehenden Ansprüche, wobei das Verbindungselement einen oder mehrere Abstandshalter umfasst zur Steuerung der Penetrationstiefe und -intensität der akustischen Druckwellen in Bezug auf den Zielbereich umfasst.

14. System nach einem der vorstehenden Ansprüche, wobei das Verbindungselement eine Befestigung zum Sichern und Verbinden der Unterlage mit dem Verbindungselement umfasst.

15. System nach einem der vorstehenden Ansprüche, wobei die leitfähige Unterlage eine Matrix von Poren umfasst, in der die bioaktive Substanz, beispielsweise ein Kolloidgel, gespeichert ist.

## Revendications

1. Système applicateur d'ondes de pression acoustique permettant d'appliquer des ondes de choc acoustiques pour faciliter l'absorption d'une substance absorbable dans une zone cible comprenant:
un générateur d'ondes de choc acoustiques (5) sélectionné parmi le groupe composé d'un générateur d'ondes de choc électrohydrauliques, un générateur d'ondes de choc électromagnétiques, un générateur d'ondes de pression radiales et un générateur d'ondes de choc piézoélectriques;
un coupleur (30) comportant un milieu conducteur permettant d'acheminer les ondes de choc acoustiques du générateur d'ondes de choc acoustiques à une zone cible; et
un patin de conduction acoustique (15) accouplé au coupleur comprenant une substance bioactive dispersible absorbée par la zone cible.

2. Système applicateur selon la revendication 1, dans lequel la substance bioactive est sélectionnée parmi le groupe consistant en une pommade, un onguent, un cosmétique, un médicament, un hydratant, un anesthésiant, un antiseptique, un antibiotique et un stérilisant.

3. Système selon l'une quelconque des revendications précédentes, dans lequel le patin est positionné pour acheminer les ondes de pression acoustiques dans un champ focal divergent vers une zone cible comme la peau.

4. Système selon l'une des revendications précédentes, dans lequel le patin comporte un matériau de criblage ou de blocage d'ondes.

5. Système selon l'une des revendications précédentes, dans lequel le patin comprend une partie bandage stérile recouvrant la gorge d'un applicateur d'ondes de choc abritant le coupleur.

6. Système selon l'une des revendications précédentes, dans lequel le patin comprend un gradient d'une pluralité de matériaux.

7. Système selon l'une des revendications 1, 2 et 4-6, dans lequel le patin est conformé pour focaliser une onde de pression acoustique.

8. Système selon l'une des revendications 1, 2 et 4-6, dans lequel le patin est conformé pour diffuser une onde de pression acoustique.

9. Système selon l'une des revendications précédentes, dans lequel le patin comporte une ou plusieurs compositions affectant les caractéristiques de focalisation d'une onde de pression acoustique.

10. Système selon l'une des revendications 1, 2, 4-6 et 9, dans lequel le coupleur est configuré pour focaliser une onde de choc vers un point focal de convergence (10) dans le patin.

11. Système selon l'une des revendications 1, 2, 4-6 et 9, dans lequel le coupleur est configuré pour focaliser une onde de choc vers un point focal de convergence dans la zone cible.

12. Système selon l'une des revendications précédentes, comprenant en outre un élément de focalisation réglable coaxialement permettant de contrôler la profondeur de pénétration des ondes de pression acoustique dans et au-delà de la zone cible.

13. Système selon l'une des revendications précédentes, dans lequel le coupleur comprend une ou plusieurs entretoises permettant de contrôler la profondeur de pénétration et l'intensité des ondes de pression acoustiques par rapport à la zone cible.

14. Système selon l'une des revendications précédentes, dans lequel le coupleur comporte un système de fixation permettant de fixer et accoupler le patin au coupleur.

15. Système selon l'une des revendications précédentes, dans lequel le patin de conduction comporte une matrice de pores stockant la substance bioactive sous forme de gel colloïdal.
